# EUROPEAN PATENT APPLICATION

(11) **EP 3 714 874 A1**
(43) Date of publication of application: **30.09.2020**
(21) Application number: 19305415.2
(22) Date of filing: 29.03.2019
(51) Int. Cl.: A61K 9/00, A61K 9/50, A61K 9/51

(54) **CAPSULE COMPRISING INSULIN-SECRETING CELLS FOR TREATING DIABETES**

(71) Applicant: Commissariat à l'Énergie Atomique et aux Énergies Alternatives, 75015 Paris cedex (FR); Université Grenoble Alpes, 38400 Saint-Martin-d'Hères (FR); Etablissement Français du Sang, 93210 La Plaine Saint Denis (FR); Wake Forest University Health Sciences, Inc., Winston-Salem, NC 27157 (US)
(72) Inventor: LABLANCHE, Sandrine, 38190 BERNIN (FR); BENHAMOU, Pierre-Yves, 38240 Meylan (FR); TUBBS, Emily, 38100 Grenoble (FR); MOISAN, Anaïck, 38420 REVEL (FR); EGELHOFER, Harald, 38700 CORENC (FR); PERSOONS, Virginie, 38330 BIVIERS (FR); RIVERA, Florence, 38054 GRENOBLE CEDEX 09 (FR); BOTTAUSCI, Frédéric, 38054 GRENOBLE CEDEX 09 (FR); PIERRON, Maxime, 38054 GRENOBLE CEDEX 09 (FR); ORLANDO, Giuseppe, Winston-Salem, North Carolina 27106 (US); TAMBURRINI, Riccardo, Winston-Salem, North Carolina 27104 (US)
(74) Representative: Lavoix

(57) **Abstract**

The application concerns a capsule comprising alginate encapsulating:
- insulin-secreting cells,
- a RGD-alginate,
- mesenchymal stem cells,
- extracellular matrix,
the process for the preparation thereof and the use thereof for treating diabetes.

## Description

The present invention concerns capsules containing insulin-secreting cells for the treatment of diabetes.

Type 1 diabetes is caused by an auto-immune destruction of beta cells leading to irreversible insulinopenia.

The management of type 1 diabetes relies exclusively on multiple daily injections of insulin. For these patients, the disease is always present in daily life, requiring glycemia measurements, calculations of carbohydrate intakes and insulin injections and impacting notably patient's quality of life. Moreover, despite intensive insulin therapy, microangiopathic complications remain a reality for diabetic patients. Moreover, a small proportion of patients develop brittle diabetes, a rare form of type 1 diabetes exposing patients to high glucose variability and severe metabolic events such as hypoglycemic coma or keto-acidosis.

Islet transplantation or cell therapy of type 1 diabetes is a technique now validated for the management of unstable diabetic patients and it exposes patients to a lower morbidity and mortality than the pancreas transplantation. Islet transplantation represents a hope for millions of type 1 diabetic patients offering the perspective of an endogenous regulation of glycemia without insulin injection. The weakness of this therapy remains the scarcity of organ source, the long term loss of islet graft functionality and the requirement for life-long immunosuppression. The extension of cell therapy of type 1 diabetes to the greatest number of patients with type 1 diabetes is conditioned by the discontinuation of immunosuppressive therapy.

Many projects focus on islet encapsulation, which provides an innovative perspective to avoid immunosuppression during transplantation. The concept of encapsulation involves applying a physical barrier between cells and the immune system.

Alginate is one of the most used polymer for cell encapsulation as it allows rapid gelling under physiological conditions. Insulin secreting human islets (also called "Islets of Langerhans") encapsulated within an alginate capsule grafted into mammals has already been reported by O'Shea et al. (O'Shea, G. M., Goosen, M. F. & Sun, A. M. Prolonged survival of transplanted islets of Langerhans encapsulated in a biocompatible membrane. Biochim. Biophys. Acta 804, 133-136 (1984)). In this study, in one of the five animals the microencapsulated islets remained viable for 365 days. Duvivier-Kali et al. reported a survival of more than 350 days for syngenic and allogenic transplantation of islets encapsulated in high-mannuronic (high-M) alginate cross-linked with BaCl₂. (Duvivier-Kali, V. F., Omer, A., Parent, R. J., O'Neil, J. J. & Weir, G. C. Complete Protection of Islets Against Allorejection and Autoimmunity by a Simple Barium-Alginate Membrane. Diabetes 50, 1698-1705 (2001)). However, BaCl₂, at concentrations greater than 5 to 10 mM, is known to be an inhibitor of potassium channels in biomembranes and therefore is not biocompatible. A major weakness identified concerns the functionality of encapsulated cells, which is limited by inadequate oxygen and nutrients delivery within the capsule after implantation.

Grafting insulin secreting human islets is really challenging, because the islets have to be able to produce and release insulin and should also remain viable in order to allow the production and release of insulin over time with an appropriate kinetic. Thus, the development of an encapsulation device should enable insulin and glucose diffusion in order to achieve optimal glucose metabolism. Ideally, an encapsulation device should also guarantee optimal oxygen and nutrient diffusion through the capsule to sustain survival and function of islet mass for the maintenance of normoglycaemia. Vascularization around the capsules should be promoted and fibrosis should be avoided.

Rejection of the transplanted islets by the host immune system should also be avoided without using immunosuppressive drugs. Indeed, the immune system reacts to any transplant, and required the use of lifelong immunosuppressors so that the patient doesn't reject the graft. In the long term, this immunosuppressive treatment is often associated with serious adverse events such as hypertension, increased susceptibility to infections and increased risk of cancer. In previous studies, some encapsulated islets required immunosuppressants to be administered. Avoiding immunosuppressants would be beneficial for the patient. Besides, immunosuppressive treatments are also costly especially considering patients have to take this treatment for all their life.

When used in vivo, the material used for the composition of the capsule should be biocompatible.

Moreover, the size of the islets/of the capsules should be controlled in order to allow: standardization and control of the quantity of insulin produced in time as well as reproducibility from one patient to another.

There is a need for implantable devices comprising islets and exhibiting all of these properties.

To this aim, according to a first object, the invention concerns a capsule comprising alginate encapsulating:
- insulin-secreting cells,
- a RGD-alginate (also named "alginate-RGD"),
- mesenchymal stem cells (MSCs), and
- extracellular matrix (ECM).

The core of the capsule contains insulin-secreting cells.

Insulin-secreting cells are chosen from insulin secreting human, multipotent stem cells able to differentiate into insulin secreting human islets, progenitor cells able to differentiate into insulin secreting human islets and mixtures thereof, and are preferably insulin secreting human islets. The insulin secreting human islets can be pancreatic islets isolated from human donors, or pancreatic islets isolated from mammal such as porcine or murine models.

The core of the capsule can consist of insulin-secreting cells.

When the insulin-secreting cells are insulin secreting human islets, the mean number of islets per capsules is generally from 0.5 to 10.0, notably from 0.75 to 5.0, typically from 0.8 to 3.0, preferably from 0.8 to 2.0, most preferably from 0.9 to 1.5, 1.0 islet per capsule being preferred. Capsules containing a single islet could maximize its viability. Two diffusion phenomena need to be considered. The diffusion through the alginate to reach the islets is the first one. The spherical shape of the capsule maximizes the surface area to volume ratios and enables a uniform diffusion between the islets and the environment outside the capsule, thus promoting improved oxygen and nutrient exchange. The second phenomenon is the diffusion through the islets. Having more than 1 islet promotes the necrosis of the cells in the center of the cluster since they form a barrier to the diffusion.

The insulin-secreting cells, RGD-alginate, mesenchymal stem cells, and extracellular matrix are dispersed within the alginate.

In the present application, "alginate" means an alginate free of RGD moieties, whereas "RGD-alginate" or "alginate-RGD" means an alginate comprising RGD moieties. Accordingly, the "alginate" and the "RGD-alginate" are two different compounds.

Alginate is used as the encapsulation material owing to its availability, cost and ease of production. Capsules obtained from alginate are advantageously resistant yet elastic. The counter ions of the alginate are generally multivalent cations, preferably chosen from calcium, barium, cobalt and mixtures thereof, and most preferably from calcium and mixtures of barium and calcium. Calcium is the most preferred counter-ion. These counter-ions are advantageously biocompatible.

In one embodiment, the alginate is a M-rich alginate (also named "high-M alginate"), i.e. an alginate wherein the ratio of mannuronic acid ("M") units versus guluronic acid ("G") units is higher than 1. Compared to G-rich alginate, M-rich alginate forms a more rigid capsule which is thus more resistant. A rigid capsule presents a major advantage regarding the administration route of the capsules. Indeed, the capsules containing the insulin-secreting cells will be administered by injection to the patient, meaning that the capsules need to resist to pressure during the implantation and also be maintained in time.

In another embodiment, the alginate is a G-rich alginate (also named "high-G alginate"), i.e. an alginate wherein the ratio of guluronic acid units versus mannuronic acid units is higher than 1. Compared to M-rich alginate, G-rich alginate has less mannuronic acid units which are triggers for cytokines but are also less elastic which is an important factor for the transplantation. The permeability of the capsule is interesting in that it allows glucose and oxygen to penetrate within the capsules, and oxygen is required for the viability of the insulin-secreting cells, and it allows insulin to get out of the capsules, i.e. the release of insulin. A porous capsule is essential, but it should not be too permeable as the capsule should protect the insulin-secreting cells within the capsules from the cytokines and antibodies produced by the immune system. The capsule should be porous to small size molecules such as oxygen, glucose and insulin but should not let in larger molecules such as cytokines or even antibodies. Accordingly, a compromise has to be chosen as regards the elasticity / the permeability of the alginate capsule.

In another embodiment, the alginate is a mixture of M-rich alginate and G-rich alginate.

The alginate generally has a molecular weight between 75 and 250 kDa. Such molecular weights allow obtaining an appropriate rigidity and porosity, even with calcium as counter-ion for the alginate.

The thickness of the alginate from the insulin-secreting cells to the surface of the capsule is preferably lower than 300 µm, notably lower than 250 µm, most preferably lower than 200 µm. This thickness is measured by microscope, namely by measuring the capsule diameter and subtracting the diameter of the insulin-secreting cells. This thickness also impacts the diffusion kinetics of the cytokines, oxygen and insulin across the capsule. The lower the thickness is, the faster the diffusion of the different components. Thicknesses higher than 300 µm do generally not allow sufficient diffusion-in of oxygen and/or diffusion-out of insulin from the insulin-secreting cells.

The capsules generally comprise from 0.5 to 4% by weight, preferably from 1.5 to 3.5% by weight, most preferably from 2.0 to 3.0% by weight of alginate. The inventors proved that the more concentrated the alginate is (within the concentrations listed above), the less porous and thus less permeable the capsule is. As explained above, a permeable capsule is required for the diffusion of oxygen, nutriments and insulin to penetrate the capsule. Accordingly, more than 4% by weight of alginate is preferably avoided. Moreover, less than 1.5% by weight of alginate generally leads to too porous capsule.

The capsule further comprises mesenchymal stem cells (MSCs), which:
- facilitate (stimulate, through the secretion of growing factors) the vascularization around the capsules and thus the oxygen and nutriments supply into the capsules,
- modulate the local immune response (MSCs secrete factors which are able to modulate macrophage activation and T cell phenotype, in the context of alloimmune responses and to modulate myeloid leukocytes and lymphocytes that are involved in the immune response through multiple mechanisms, including cell-cell contact and indirect contact through cytokines and signaling molecules) and
- may also improve graft function and mitigate the fibrotic foreign body response. MSCs can be isolated from adipose tissue, bone marrow, umbilical cord or derived from pluripotent cells.

MSCs are unable to naturally adhere to the alginate of the capsule. The capsule thus further comprises a RGD-alginate, which consists in an alginate grafted with RGD moieties (i.e. arginine-glycine-aspartic acid sequence). RGD peptides specifically conjugate integrins in cell membranes. The RGD-alginate thus increases the viability of the MSCs by facilitating MSCs adherence, proliferation and spreading. The RGD-alginate has generally a minor or no effect on the viability of the encapsulated insulin-secreting cells themselves. But the RGD-alginate increases the viability of the MSCs, which increases the viability of the insulin-secreting cells. There is a synergistic effect with the combined use of a RGD-alginate and MSCs, which leads to an increased viability of the insulin-secreting cells compared to capsules with only MSCs, or only RGD-alginate.

The alginate of the RGD-alginate can be a M-rich alginate, a G-rich alginate or a mixture of M-rich alginate and G-rich alginate. The RGD:Alginate ratio of the RGD-alginate is preferably from 0,005 to 0,020 µmol/mg, most preferably from 0.012 µmol/mg to 0.013 µmol/mg. The degree of peptide substitution within the RGD:Alginate is preferably from 0.1 to 0.3%, most preferably from 0.239% to 0.380%.

The capsule further comprises extracellular matrix (ECM), preferably pancreatic extracellular matrix, most preferably obtained from the human pancreas. The rationale for doing so is that the ECM recapitulates the niche of the insulin-secreting cells, restores their native microenvironment which is disrupted during the isolation process, provides critical signaling for insulin-secreting cells, to ultimately enhance viability, function and lifespan.

The capsules preferably comprise:
- from 0.1 to 1.5 mg/mL, most preferably from 0.2 of 1.0 mg/mL, of RGD-alginate, versus the total volume of alginate, RGD-alginate, mesenchymal stem cells and extracellular matrix (i.e. without taking into account the volume of insulin-secreting cells), and/or
- from 0.01 to 1.0 mg/L, most preferably from 0.05 to 0.5 mg/L, of ECM versus total volume of alginate, RGD-alginate, mesenchymal stem cells and extracellular matrix (i.e. without taking into account the volume of insulin-secreting cells), and/or
- from 10 to 10000 MSCs, notably from 20 to 5000 MSCs, preferably from 25 to 500 MSCs, most preferably from 50 to 150 MSCs per capsule (i.e. by taking into account all the components thereof, including the insulin-secreting cells).

Less than 1.0 mg/L of ECM and/or from 0.1 to 1.5 mg/mL of RGD-alginate generally allows maintaining a good viability of the cell lines in contact with the capsules. From 0.05 to 0.5 mg/L of ECM, preferably around 0.1 mg/L of ECM allows maintaining at best the phenotype of the MSCs.

From 10 to 500 MSCs, most preferably from 50 to 150 MSCs allow an appropriate insulin secretion from the capsules.

The capsule can comprise further cells and/or materials and/or compounds. For example, it can comprise hyaluronic acid, which can help the insulin-secreting cells.

The surface of the capsule is preferably at least partly coated with a zwitterionic polymer. A classic host immune reaction to a foreign body includes fibrosis, which can significantly limit the performance and life span of the implanted capsules. The surface properties of implanted capsules play an important role in modulating the fibrotic response. In this context, implanted capsules can trigger anti-immune reaction, including acute/chronic inflammation, a foreign body reaction and formation of a fibrotic capsule of collagen around the capsules. To limit the fibrotic foreign body response, the surface of the capsule is at least partly coated with a zwitterionic polymer, which naturally exhibits anti-fouling properties. At least partly coated means that either only a part (or some parts) of the surface is(are) coated, while another (or others) is(are) not, or the whole surface of the capsule is coated with the zwitterionic polymer. Zwitterionic polymers have already been used for coating the surface of surgical devices, but the inventors are not aware of their use for coatings capsules with encapsulated cells. Zwitterionic polymer is deposited on the capsule surface according to a surface modification process, which preserves the alginate.

The zwitterionic polymer is preferably biocompatible, namely it does not present any toxicity neither for the components of the capsule not for the surroundings thereof, i.e. for the host on which the capsule is implanted.

The zwitterionic polymer preferably has a water solubility at 25°C higher than 1 g/L, most preferably higher than 3 g/L.

The zwitterionic polymer preferably has:
- a net positive charge (thus allowing its grafting on the negatively charged alginate), and/or
- a mass-average molar mass from 25 000 to 500 000 g/mol, notably from 40 000 to 200 0000 g/mol, most preferably from 50 000 to 150 000 g/mol, as such molar masses limit its potential diffusion into the alginate.

The zwitterionic polymer generally comprises positively charged units, units grafted with zwitterionic moieties (which provide antifouling properties) and optionally uncharged units, such as acetylated units. The zwitterionic polymer preferably typically:
- a quantity of positively charged units of about 60 to 70%,
- a quantity of uncharged units of lower than 10%,
- a quantity of units grafted with zwitterionic moieties of about 20 to 30%,
wherein the quantities are expressed as the number of units per 100 units within the zwitterionic polymer. The zwitterionic moieties provide antifouling properties.

Determining the net positive charge of the zwitterionic polymer generally required determining the quantity of positively charged units. This can be done by determining the quantity of uncharged units and of units grafted with zwitterionic moieties. For example, when the polymer is a chitosan wherein the uncharged units are acetylated units:
- the initial deacetylation degree of the chitosan (generally determined by NMR) "X1" is determined,
- the grafting degree of zwitterionic moieties (general determined by combining the measurements of Total Organic Carbon (TOC) content and UV-VIS and FTIR spectroscopies) "X2" is determined,
- the net positive charge is 100-X1-X2.

Preferably, the zwitterionic polymer is chitosan on which zwitterionic moieties have been grafted. Chitosan is an excellent, safe and biocompatible biopolymer for biomedical applications. Moreover, it is a positively charged derivative which interacts electrostatically with negatively charged alginate, so that the surface of the capsule is at least partly coated with a zwitterionic chitosan. The embodiments described above apply. For example, the quantity of zwitterionic moieties grafted on the chitosan is preferably of about 20 to 30%.
(propargyloxy)di(ethoxy)ethylphosphorylcholine can be used as zwitterionic moiety.

The mean diameter of the capsule is typically from 50 µm to 5 mm, preferably from 50 µm to 3 mm, to 75 to 950 µm, most preferably from 100 to 800 µm. Capsules of such sizes can advantageously contain a single islet. The insulin secreting human islets which are encapsulated generally have a mean diameter from 50 µm to 500 µm. The bigger the islets are, the less they produce insulin. Islets measuring less than 50 µm are generally small islets as well as debris which are not able to produce enough insulin.

The aspect ratio (ratio of its longer side to its shorter side) of the capsule is generally lower than 1.15. The capsule is generally not fully spherical, i.e. its aspect ratio is generally higher than 1.01.

The capsules are monodisperse, i.e. the coefficient of variation (CV) thereof (ratio of the standard deviation to the mean diameter) is lower than 5%, typically from 3 to 4%. The monodispersity is advantageous in that islets having about the same diameter are encapsulated within a capsule wherein the alginate thickness from the islet to the surface of the capsule is the same for all the capsules. Thus two different capsules are able to produce about the same quantity. In other words, capsules with
- a narrow size distribution
- a controlled number of low size islets
are advantageous to better control the insulin production.

The mean diameter and aspect ratio are preferably measured by taking pictures using a camera mounted on a microscope of at least 10 capsules and analyzing the picture with ImageJ software. This measurement method is advantageous in that it takes into account the alginate, which is not visible via laser diffraction as it is an aqueous medium. The measure is generally done at room temperature, i.e. at 25°C.

The capsules according to the invention are advantageously biocompatible, and allow the production of insulin with appropriate kinetics and survival of insulin-secreting cells by modulating the fibrotic response, in order to reach generally 6-months, notably one year, or even 2-years of insulin injection free treatment, without the need for immunosuppressants.

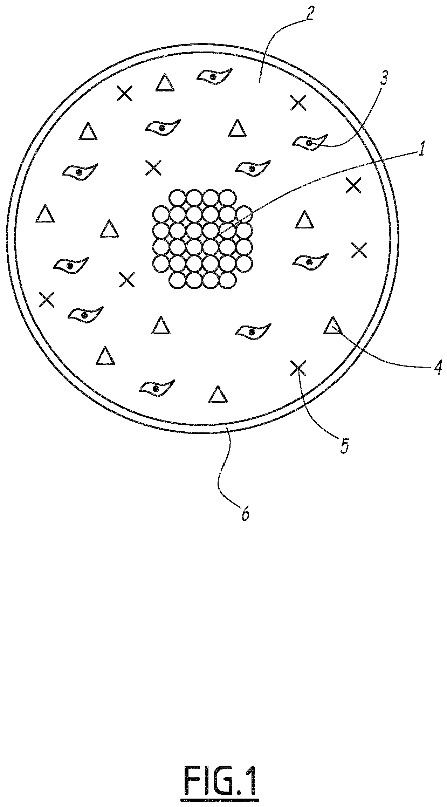

Figure 1 illustrates a cross-section of a capsule according to the invention.

According to a second object, the invention concerns a process for preparing the capsules described above, comprising the steps of:
a) providing an encapsulation solution which is an aqueous solution comprising alginate, RGD-alginate, ECM, MSCs and insulin-secreting cells,
b) encapsulating insulin-secreting cells by bringing droplets of the encapsulation solution in contact with an aqueous solution comprising multivalent cations able to react with alginate, to obtain at least a capsule comprising alginate encapsulating insulin-secreting cells, the RGD-alginate, mesenchymal stem cells, and extracellular matrix,
c) optionally at least partly coating the surface of said capsule by a zwitterionic polymer. Step a) typically comprises the sub-steps consisting of:
   a1) providing a first aqueous solution comprising alginate, RGD-alginate and ECM,
   a2) providing a second aqueous solution comprising MSCs and insulin-secreting cells,
   a3) bringing into contact the first and second solutions in order to obtain an encapsulation solution which is an aqueous solution comprising alginate, RGD-alginate, ECM, MSCs and insulin-secreting cells.

The desired number and size of insulin secreting-cells to be encapsulated determines the number of capsules to be produced, and hence determines the volume of first aqueous solution to be used and the number of MSCs to be used.

When used in vivo, the material used for the composition of the capsule should be biocompatible. This requires that the components used to prepare it are also biocompatible. Accordingly, components with low levels of endotoxins are preferred.

The process can comprise, before step a1), a step a1') of preparing the first aqueous solution comprising alginate, RGD-alginate and ECM.

Step a1') typically comprises the sub-steps of:
a1'i) preparing an aqueous solution comprising alginate,
a1'ii) preparing an aqueous solution comprising RGD-alginate,
a1'iii) preparing an aqueous solution comprising ECM,
a1'iv) mixing the aqueous solutions obtained at steps a1'i), a1'ii) and a1'iii) to obtain the first aqueous solution comprising alginate, RGD-alginate and ECM.

Preferably, the aqueous solution comprising alginate prepared at step a1'i) has a viscosity from 0.01 to 30 Pa.s, preferably 0.1 to 25 Pa.s at a temperature of 20°C and at a shear of 0.01 s⁻¹. The viscosity is measured using a rheometer.

Such highly viscous aqueous solutions are usually disregarded to encapsulate cells, because encapsulation is difficult: the high viscosity hinders the ability of the aqueous solution to form droplets and capsules. High concentrated alginate has been chosen because it has been shown in the literature that the pores size of the alginate decreases as the concentration increases. The inventors managed to obtain capsules, and even monodisperse capsules, with highly viscous alginate based shell thanks to the use of Microfluidic Flow-Focusing Device (MFFD) (which is unexpected in the field of alginate based shell encapsulating cells) or of centrifugal microfluidics, such as described in Cheng et al. Applied Materials Today 13(2018) 116-125 or Maeda et al., Advanced Materials, 2012, 24, 1340-1346. Accordingly, the capsules are preferably obtained by using a Microfluidic Flow-Focusing Device (MFFD) or centrifugal microfluidics. These techniques are also advantageous in that:
- the duration for preparing the capsules is generally lower than with other methods, thus maximizing the ability of the encapsulated cells, and/or
- less protrusions are generally observed on the capsules when these methods are used.

An ID-test can be performed for the RGD-alginate used in the process (in particular for step a1'ii)), said test being positive. The appearance of the RGD-alginate is generally a white powder. The RGD:Alginate ratio of the RGD- alginate is preferably from 0,005 to 0,020 µmol/mg, most preferably from 0.012 µmol/mg to 0.013 µmol/mg. The degree of peptide substitution is preferably from 0.1 to 0.3%, most preferably from 0.239% to 0.380%. The endotoxin level of the RGD-alginate is preferably lower than 25 EU/gram. Elemental impurities within the RGD-alginate are generally lower than 10 ppm. Step a1 (and step a1'ii)) is preferably preceded by steps of providing the RGD peptide, preferably having the features described above, then microfiltering the RGD peptide, preferably at 0.22 µm, then freeze drying the RGD-peptide.

The ECM used to implement the process and in particular step a1'iii) is preferably obtained through a water-based decellularization method that advantageously allows for a thorough preservation of pancreatic matrisome and avoids the use of toxic ionic and non-ionic detergents commonly used in decellularization processes.

The process can comprise, before step a2), a step a2') of preparing the second aqueous solution comprising MSCs and insulin-secreting cells. Step a2') typically comprises the sub-steps of:
a2'i) detaching MSCs from their culture surface using trypsine, centrifugation and resuspension of MSCs to obtain a solution comprising MSCs,
a2'ii) centrifuging the solution comprising MSCs and removing the supernatant to obtain a pellet,
a2'iii) resuspending the pellet in a calcium enriched physiological serum to obtain a suspension of MSCs,
a2'iv) counting the MSCs within this suspension, then
a2'v) taking the appropriate volume of suspension to obtain the amount of MSCs required, then
a2'vi) centrifuging the suspension and removing the supernatant to obtain a pellet, then
a2'vii) resuspend the remaining pellet in alginate buffer solution to obtain an alginate solution comprising MSCs,
a2'viii) adding and mixing insulin-secreting cells within the alginate solution comprising MSCs to obtain an alginate solution comprising MSCs and insulin-secreting cells.

The process comprises step b) of encapsulating the insulin-secreting cells by bringing droplets of the encapsulation solution in contact with an aqueous solution comprising multivalent cations able to react with alginate, to obtain at least a capsule comprising alginate encapsulating insulin-secreting cells. Bringing into contact droplets of the encapsulation solution with an aqueous solution comprising multivalent cations able to react with alginate leads to cross-linking of the alginate and thus to the formation of alginate gel around the insulin-secreting cells. Alginate chains aggregates and forms a physical gel in the presence of the multivalent cations able to react with alginate.

The multivalent cations are generally calcium, cobalt and/or barium ions. The aqueous solution is generally a CaCl₂, CoCl₂ and/or BaCl₂ aqueous solution.

The process preferably comprises, between steps a) and b), a step α) consisting of forming droplets of the encapsulation solution by introducing the encapsulation solution within a Microfluidic Flow-Focusing Device (MFFD). This allows producing monodispersed capsules with a controlled diameter. This is usually not possible when other encapsulation methods such as emulsion or dripping technologies are used. This also allows using a highly viscous alginate, which is difficult with other techniques.

When the surface of the capsule is at least partly coated with a zwitterionic polymer, the process comprises step c) of at least partly coating the surface of the capsule with a zwitterionic polymer. Step c) is typically carried out by immersing the capsule obtained after step b) within an aqueous solution of zwitterionic polymer, preferably for a duration less than 4 min, most preferably for about 3 min.

The process can comprise, before step c), a step c') of preparing the zwitterionic polymer comprising notably a step of grafting zwitterionic moieties to a polymer. The zwitterionic moieties can be grafted thanks to a linker. For example, when the zwitterionic polymer is chitosan grafted by (propargyloxy)di(ethoxy)ethylphosphorylcholine moities, 4-azidobutyrate N-hydroxysuccinimide can be used as compound bearing anchoring groups to form the linker.

The process can comprise, between steps b) and c), and/or after step c), a step β) of washing the capsule(s), preferably with a calcium enriched physiological serum. Step β) can optionally be repeated.

The obtained capsule in a calcium-enriched serum can easily be stored and/or transported.

According to a third object, the invention concerns the capsule described above for its use in the treatment of diabetes, preferably type 1 and/or insulin-treated type 2 diabetes. The invention also concerns a method for treating diabetes, preferably type 1 and/or insulin-treated type 2 diabetes, comprising administering to a mammal or a patient in need thereof at least a capsule as described above. The invention also concerns a method for treating diabetes, preferably type 1 and/or insulin-treated type 2 diabetes, comprising implanting to a mammal or a patient in need thereof at least a capsule as described above. The invention also concerns the use of the capsule as described above for treating diabetes, preferably type 1 and/or type 2 diabetes. The treatment is preferably free of immunosuppression treatment, i.e. no immunosuppressor need to be administered previously, simultaneously or subsequently to the capsules.

The enclosed figures and the examples hereafter illustrate the invention.

### FIGURES

Figure 1: Scheme illustrating a cross-section of a capsule according to the invention
   1: insulin-secreting cells such as islets
   2: alginate
   3: MSCs
   4: ECM
   5: RGD-alginate
   6: zwitterionic polymer coating
Figure 2: Slide scanner image of the tissue of a rat wherein capsules without zwitterionic chitosan coating have been grafted.
   Fibrosis: thin black arrows
   Capsules: black asterisks
   Healthy tissue: large black arrows
Figure 3: Slide scanner image of the tissue of a rat wherein capsules with a zwitterionic chitosan coating have been grafted.
   Fibrosis: thin black arrows
   Capsules: black asterisks
   Healthy tissue: large black arrows

### EXAMPLES

In the examples below were used:
- PRONOVA SLM100 (abbreviated "SLM" below) was used as alginate,
- NOVATACH™ MVG GRGDSP (GRGDSP-coupled high G high MW alginate) as RGD-alginate,
- Ultra-pure ECM
- Human MSCs
- as zwitterionic polymer was used zwitterionic chitosan.

The zwitterionic chitosan was obtained as follows:
chitosan with a mass-average molar mass of about 50 000-150 000 g/mol was deacetylated et a deacetylation degree higher than 10% (expressed as the number of monomer units) and grafted with 20-30% (expressed as the number of moieties grafted for 100 deacetylated anhydroglucose units) of (propargyloxy)di(ethoxy)ethylphosphorylcholine. The zwitterionic moieties of the zwitterionic chitosan were obtained by grafting (propargyloxy)di(ethoxy)ethylphosphorylcholine moieties thanks to a 4-azidobutyrate N-hydroxysuccinimide.
The obtained zwitterionic chitosan contained 60-70% of positively charged monomer units, 20-30% of units grafted with zwitterionic moieties and not more than 10% of acetylated (= uncharged) units. The percentage of positively charged monomer units for zwitterionic chitosan chains was of about 60-70%.
The zwitterionic chitosan was purified in aqueous solution and stored in aqueous solution after the synthesis. The zwitterionic chitosan is soluble in water (with a water solubility higher than 3 g/L at 25°C) only if it is not freeze dried after the synthesis. If it is freeze dried, the dried zwitterionic chitosan becomes insoluble.

The ECM used in the examples was obtained as follows. The ECM was obtained through a water-based decellularization method that advantageously allows for a thorough preservation of pancreatic matrisome and avoids the use of toxic ionic and non-ionic detergents commonly used in decellularization processes.

Pancreatic matrices are obtained as follows:
Pancreata not deemed suitable for transplantation and allocated for research purposes were obtained and processed. Organs are frozen and stored in sterile conditions at before being used to produce ECM. Organ donors are screened for infectious diseases relevant to humans. All organs used as a source of ECM are clinically safe.

Surgical preparation of the pancreas: Frozen pancreases are thawed. Human pancreata are removed from all extra pancreatic tissue. The surgically prepared pancreatic parenchyma is dissected in approximately 1 cm³ cubes and washed respectively in a Betadine and Pen-Strep solution for 15 minutes before undergoing the process of decellularization. Pancreatic tissue may or may not undergo a process of delipidation before the decellularization process. Delipidation is obtained through no more than 5 incubations of the pancreatic tissue in methanol-chloroform solution through a serial incubations of the tissue in ethanol.

Decelullarization process: *(1)* diced pancreatic tissue is placed in a sterile container with 1L of sterile deionized H₂O and placed shaking for 24h at 200 rpm at 4°C. *(2)* In the second day of decellularization the tissue undergoes an enzymatic digestion with a DNAse (5%)/MgCl solution (1L) at adjusted pH 7.4 at 37°C on a shaker at 100 rpm for 6h; the tissue is then incubated in an EDTA-Trizma based solution (1L) on a shaker at 200 rpm for 18h at 4°C; *(3)* In the third day of decellularization the tissue is washed in sterile deionized H₂O for 24h.

The obtained scaffold is frozen at -80°C, lyophilized (Freeze-Dryer Technique) and cryomilled to obtain a fine ECM powder.

The ECM powder is solubilized at room temperature at constant stirring in a pepsin-HCI solution for no more than 48 hours. The solution pH is re-equilibrated to pH=7.4 with NaOH to irreversibly inactivate the pepsin. The so-obtained solubilized ECM is centrifuged and the supernatant collected. The supernatant collected from the solubilized ECM is frozen at -80°C and lyophilized in order to obtain a fine powder, now called UltraPure Soluble ECM. The UltraPure is vialed in glass ampoules, sealed and subsequently sterilized for research-clinical use.

### Example 1: Effect of the concentration of alginate on the permeability of the obtained capsule

The microcapsules are produced as mentioned in Example 7.1 (free of zwitterionic chitosan coating). The biopolymer is made only of 1.5%w/w of SLM for the first case and 2.5%w/w of SLM for the second.

We exposed capsules to different Dextran (Sigma-Aldrich) ranging from 10 kDa to 2000 kDa and IgG at 0.033 mg/mL. We mixed glass beads (Polysciences Inc. 355-420µm #18905) with the capsules as intern controls. We put each condition in a well of 12-well-plate. The plates were covered with aluminium and stirred with a STUART Mini Gyro-rocker SSM3 for 72 hours at 4°C at 70 rpm. Images were acquired with a Leica TCS SP8 inverted laser scanning confocal microscope (Leica Microsystems, Wetzlar, Germany) equipped with an objective HC PL Fluotar x10/0.30. Laser excitation was 488 nm and fluorescence emission was collected by a spectral detector HYD GaAsP at 500-550 nm. We then measured the greyscale level inside the capsules with ImageJ on the acquired images and deducted an absolute level of fluorescence by comparing with the background of the images.

The absolute concentration of the dextran, IgG or glass beads released within the solution are provided in table 1. The higher the concentration value, the higher the capsule permeability.

**Table 1: Permeability of 1.5%wt or 2.5%wt SLM capsules encapsulating different materials**

| Encapsulated material | Permeability obtained with capsules with 1.5%wt of SLM | Permeability obtained with 2.5%wt of SLM |
|---|---|---|
| Dextran 10 | 0.864 +/- 0.040 | 0.858 +/- 0.047 |
| Dextran 20 | 0.699 +/- 0.067 | 0.557 +/- 0.064 |
| Dextran 40 | 0.521 +/- 0.044 | 0.435 +/- 0.043 |
| Dextran 70 | 0.563 +/- 0.056 | 0.424 +/- 0.037 |
| Dextran 150 | 0.499 +/- 0.056 | 0.338 +/- 0.040 |
| Dextran 250 | 0.347 +/- 0.050 | 0.234 +/- 0.043 |
| Dextran 500 | 0.232 +/- 0.045 | 0.160 +/- 0.035 |
| Dextran 2000 | 0.112 +/- 0.029 | 0.043 +/- 0.014 |
| IgG | 0.515 +/- 0.101 | 0.223 +/- 0.058 |
| Glass bead | 0.015 +/- 0.008 | 0.018 +/- 0.009 |

The results show that the permeability depends on the concentration of alginate. The SLM 2.5%w/w is less permeable than the 1.5%w/w alginate. Nevertheless, the cut-off for the SLM 2.5%w/w is for Dextran2000 (2000kDa) where the value is comparable to the glass beads. From these results, the concentration of 2.5%w/w has been selected.

In the examples hereafter, capsules comprising 2.5%wt SLM were used.

### Example 2: Effect of MSCs

### 2.1. On islet viability

Rat islets viability co-cultured with 0, 500 or 750 MSC/islet then exposed or not to cytokines (n=7) for 24h. The viability assays were made with rat islets in co-culture with different ratios of adhering MSCs: no MSCs, 500 MSCs/rat islet, 750MSCs/rat islet. Islet viability was determined by confocal microscopy using both SYTO13 (excitation/emission: 488/509 nm) and propidium iodide (PI) (excitation/emission: 493/636 nm) stainings. Briefly, cells were incubated with SYTO13 for 15 minutes and then with PI for 1 minute. Stained cells were observed by confocal microscopy (CLSM) to discriminate alive and dead cells. On the one hand, the cell-permanent SYTO13 green fluorescent nucleic acid stain exhibits green fluorescence upon binding to nucleic acids of living cells. On the other hand, PI is an intercalating agent exhibiting red fluorescence, commonly used for identifying dead cells in a population. Staining with both SYTO13 and PI enables quantification and calculation of a percentage of viable cells represented by the number of green stained nuclei normalized by the total number of nuclei (green + red nuclei). The results are provided at table 2.

**Table 2: Rat islets viability co-cultured with or without MSC**

| | Viability (%) | |
|---|---|---|
| | Basal conditions | Stress conditions |
| rat islets | 73.2 ± 3.57 | 49,4 ± 2.64 |
| 500MSCs/rat islet | 74.8 ± 4.02 | 57,4 ± 2.96 |
| 750MSCs/rat islet | 80.3 ± 5.25 | 62.2 ± 2.92 |

### 2.2. On Stimulation index (SI)

Functionality of islets in the above mentioned co-culture conditions was measured with a standard glucose stimulation insulin-secretion test. Cells were pre-incubated in 2 mL of a sub-stimulatory glucose solution (KREBS buffer supplemented with 2.8 mM glucose). After 1h, incubation medium was collected and thrown. Cells were incubated for one additional hour in 2 mL of a sub-stimulatory glucose solution (KREBS buffer with 2.8 mM glucose). After 1h, the incubation medium was collected and stored at - 80°C. Cells were then incubated in 2 ml of stimulatory glucose solution (KREBS buffer supplemented with 16.7 mM glucose). After an additional hour, the incubation medium was collected and stored at -80°C. For the insulin content measurement, acid-ethanol was added to the culture and stored at - 20°C overnight and then at -80°C. Results are presented in table 3.

Stimulation index (SI) is widely used as a parameter that reflects the insulin releasing function of islets.

**Table 3: Stimulation index of rat islets under cytokine exposure and co-cultured with or without MSC**

| | | Stimulation index (fold) |
|---|---|---|
| basal | rat islets | 1 ± 0.10 |
| | rat islets | 0.36 ± 0.05 |
| stress conditions | 500MSCs/rat islet | 0.89 ± 0.21 |
| | 750MSCs/rat islet | 0.62 ± 0.17 |

Table 4 below provides the stimulation indexes of human islets in basal conditions.

**Table 4: Stimulation index of human islets under cytokine exposure and co-cultured with or without MSC**

| | Stimulation index (fold) |
|---|---|
| human islets | 1 ± 0.30 |
| 150MSCs/human islet | 1,40 ± 0.50 |

| | |
|---|---|
| n = 3 | |

**Table 5: Stimulation index of human islets under cytokine exposure and co-cultured with or without MSC**

| | Stimulation index (fold) | |
|---|---|---|
| | Basal conditions | Stress conditions |
| human islets | 1 ± 0.27 | 0,64 ± 0.22 |
| 150MSCs/human islet | 1 ± 0.18 | 1,03 ± 0.34 |
| 500MSCs/human islet | 1 ± 0.3 | 0,88 ± 0.57 |
| 750MSCs/human islet | 1 ± 0.22 | 0,97 ± 0.25 |

| | | |
|---|---|---|
| n=3-5 different donors. | | |

A ratio of 750 MSCs/islet has been identified to enhance rat islet viability and a ratio of 500 MSCs/islet and 750 MSCs/islet to enhance rat islet functionality under cytokine exposure. This ratio was further confirmed with human islets in co-culture with different ratios of MSCs. Nevertheless, considering the islets occupation rate as well as the biomaterials occupation rates, such a ratio cannot be encapsulated. The CEA calculated the maximal number of MSCs that could possibly be inserted into the capsules and the ratio of 150 MSCs/islet has been determined. In human islets, 150 MSCs/islet seems to improve insulin secretion in basal conditions as well as under cytokine exposure. These experiments have to be confirmed with more human islets, as it is currently the limiting factor.

### Example 3: Effect of the presence and concentration of RGD-alginate within the capsule

The microcapsules are produced as specified in example 7.1 (free of zwitterionic chitosan coating). The biopolymer is composed of alginate 2.5%w/w, 150 MSCs and the RGD-alginate concentration as mentioned in the first column of the table 6. The starting concentration and volume of each involved components are adjusted proportionally to the procedure described in example 7.1.

### 3.1. Effect on MSCs viability

**Table 6: Viability of MSCs within the capsules containing or not RGD-alginate.**

| | MSCs viability (%) |
|---|---|
| Naked MSCs (adhering, not within capsules) (control) | 95.1 ± 0.50 |
| MSCs within the capsule without RGD-alginate (reference) | 71.97 ± 11.20 |
| MSCs within capsule with 0.2 mg/L of RGD-alginate | 68.5 ± 5.50 |
| MSCs within the capsule with 0.5 mg/L of RGD-alginate | 65.8 ± 6.66 |

| | |
|---|---|
| The experiments were performed 3 times with 10 000 events analyzed each time. | |

The table shows the percentages of viability of MSC by the subtraction of dead cells to the total number of cells screened and evidences a reduction in MSCs viability when encapsulated but unchanged with the biomaterials. The encapsulation process itself but not the biomaterials impacts negatively MSCs viability. Biomaterials do not evidence particular toxicity on MSC.

### 3.2. Effect on inflammation

Interleukin-6 (IL-6) is a cytokine involved in inflammation and infection responses. To determine the effect of RGD, MSCs' secretion capacity was analyzed by measuring by ELISA the expression of IL-6 in the supernatants of adhering MSCs ("naked MSCs") and after encapsulation with the reference capsule, and with RGD (at 0.2 and 0.5 mg/mL). As shown in table 7, RGD had no effect on IL-6 secretion.

**Table 7: IL6 produced depending on the presence and concentration of RGD-alginate within the capsule.**

| | IL-6 produced (pg/ml / mg/ml) |
|---|---|
| Naked MSCs (adhering, not within capsules) (control) | 6267.0 ± 2516.8 |
| MSCs within the capsules without RGD-alginate (reference) | 4768.2 ± 1500.8 |
| MSCs within the capsules with 0.2 mg/L of RGD-alginate | 5572.3 ± 1540.1 |
| MSCs within capsules with 0.5 mg/L of RGD-alginate | 3371,7 ± 1250.8 |

### 3.3. Effect on vascularization

Vascular endothelial growth factor (VEGF) is a signal protein produced by cells that stimulates the formation of blood vessels.
To determine the effect of RGD, MSCs' secretion capacity was analyzed by measuring by ELISA the expression VEGF in the supernatants of adhering MSCs ("naked MSCs") and after encapsulation with the reference capsule, and with RGD (at 0.2 and 0.5 mg/mL). As shown in the table 8, RGD had no effect on VEGF secretion.

**Table 8: VEGF produced depending on the presence and concentration of RGD-alginate within the capsule.**

| | VEGF produced |
|---|---|
| Naked MSCs (adhering, not within capsules) (control) | 671,8 ± 267.0 |
| MSCs within the capsules without RGD-alginate (reference) | 705.0 ± 227.0 |
| MSCs within the capsules with 0.2 mg/L of RGD-alginate | 655.2 ± 150.4 |
| MSCs within the capsules with 0.5 mg/L of RGD-alginate | 564.0 ± 130.5 |

These results demonstrate that RGD-alginate used within the capsules at the tested concentration does not have any effect on IL-6 and VEGF secretion factors.

### 3.4. Not encapsulated alginate-RGD effect on different cell lines viability

HEK293, A549 and Jurkat cell lines were used to evaluate the effect of alginate-RGD on cell viability. HEK293 cell line is derived from human embryonic kidney cells and is used as model of epithelial cell line. A549 is an adenocarcinoma human alveolar cell line and is recognized as a Type II pulmonary epithelial cell model. Jurkat cell line was used as a model of human T lymphocyte. Cell lines were in contact with the alginate-RGD for 24 hours (in contact but not encapsulated) during their growth phase. MTS colorimetric assay was used to measure the viability of the cells. In metabolically active cells, mitochondrial dehydrogenases reduce a tetrazolium compound into a colored product and the number of viable cells is determined after 24 h of sample addition. Triton x-100 is used as a positive control. The results are provided at table 9. Alginate-RGD did not affect the cell viability in concentrations lower than 0.6 mg/mL. With concentrations between 1.2 mg/mL and 2.5 mg/mL the viability is around 50 - 70%. However, cells treated with the maximum concentration of 5 mg/mL of alginate-RGD showed a different response depending on the cell line. Jurkat cells viability was lower than 20%, while for A549 and HEK293 the viability was around 40%.

**Table 9: cells viability in contact with alginate-RGD at different concentrations. The results are representative of three independent experiments. The percentage of cell viability was normalized to cells without alginate-RGD.**

| concentration of alginate-RGD within the capsules (mg/mL) | A549 viability (%) | HEK293 viability (%) | JURKAT viability (%) |
|---|---|---|---|
| 5 | 43.17 ± 3.11 | 39.60 ± 12.47 | 20.65 ± 3.59 |
| 2.5 | 76.50 ± 2.43 | 69.44 ± 29.07 | 37.15 ± 1.84 |
| 1.25 | 90.54 ± 2.11 | 75.58 ± 10.34 | 63.07 ± 2.01 |
| 0.63 | 95.03 ± 2.93 | 87.02 ± 9.77 | 76.31 ± 4.48 |
| 0.31 | 99.40 ± 4.13 | 99.47 ± 2.14 | 85.92 ± 1.04 |
| 0.16 | 110.29 ± 4.28 | 105.56 ± 5.38 | 101.59 ± 0.17 |
| 0.08 | 104.87 ± 6.44 | 104.69 ± 8.51 | 105.11 ± 1.29 |

Except if otherwise stated, all the other examples were carried out with capsules comprising 0.5 mg/mL of alginate-RGD as this concentration does not affect the viability of the cell lines tested.
The effect of RGD-alginate on human islets viability and functionality is shown at tables 10 and 11.

**Table 10: Human islets viability in contact with alginate-RGD at different concentrations.**

| | Viability (fold) |
|---|---|
| Human islets (not within capsules, controls) | 1.00 ± 0.18 |
| Human islets within capsule without RGD-alginate (reference) | 0.78 ± 0.12 |
| Human islets within capsule with 0.2 mg/ml of RGD-alginate | 0.95 ± 0.19 |
| Human islets within capsule with 0.5 mg/ml of RGD-alginate | 1.01 ± 0.16 |

The results are representative of three independent experiments. The percentage of Human islets viability was normalized to Human islets without alginate-RGD.

**Table 11: stimulation index of human islets under RGD-alginate exposure**

| | Stimulation index (SI) |
|---|---|
| Human islets (not within capsules, controls) | 3.1 ± 1.11 |
| Human islets within capsule without RGD-alginate (reference) | 4.0 ± 1.32 |
| Human islets within capsule with 0.2 mg/ml of RGD-alginate | 4.0 ± 2.10 |
| Human islets within capsule with 0.5 mg/ml of RGD-alginate | 5.25 ± 1.84 |

The capsules with all the different concentrations tested are functional when compared to the reference. A tendency indicates that insulin secretion could even be slightly improved by RGD 0.5mg/ml +MSCs (n=6 different donors).

### Example 4: Effect of the presence and concentration of MSC within the capsule

The microcapsules are produced as mentioned in Example 7.1 (free of zwitterionic chitosan coating). The biopolymer is made only of 2.5%w/w of SLM.

**Table 12: Viability of islets in culture and in co-culture with MSCs (white) compared to encapsulated islets in the reference capsule + MSCs (n=3-7, different donors), p < 0.05.**

| | Viability (%) |
|---|---|
| Naked islets | 76.85 |
| Islets+MSCs | 77.62 |
| 2.5% Alginate (reference) | 55.49 |
| Reference+MSCs | 57.99 |

**Table 13: Functionality of islets encapsulated with the different biomaterials measured with a standard glucose stimulation insulin-secretion test. Human islets encapsulated in the reference capsule or reference capsule + MSCs exhibite similar stimulation index to naked islets (n=6 different donors).**

| | Stimulation index |
|---|---|
| Naked islets | 3.11 |
| 2.5% Alginate (reference) | 3.97 |
| Reference+MSCs | 3.53 |

Human islets encapsulated in the reference microcapsule with MSCs exhibit similar viability than reference capsule, and remain as functional as the reference capsule filling the in vitro quality control defined in the bioactive microcapsule specifications file.

Except if otherwise stated, all the other examples were carried out with capsules comprising 150 MSCs/capsule.

### Example 5: Effect of ultrapure soluble pancreatic extracellular matrix (ECM)

### 5.1. Not encapsulated ECM effect on different cell lines viability

HEK293, A549 and Jurkat cell lines were used to evaluate the effect of ECM on cell viability. HEK293 cell line is derived from human embryonic kidney cells and is used as model of epithelial cell line. A549 is an adenocarcinoma human alveolar cell line and is recognized as a Type II pulmonary epithelial cell model. Jurkat cell line was used as a model of human T lymphocyte. Cell lines were in contact with the ECM for 24 hours (in contact but not encapsulated) during their growth phase. MTS colorimetric assay was used to measure the viability of the cells. In metabolically active cells, mitochondrial dehydrogenases reduce a tetrazolium compound into a colored product and the number of viable cells is determined after 24 h of sample addition. Triton x-100 is used as a positive control. The results are provided at table 14.

**Table 14: Cells viability in contact with ECM at different concentrations. The results are representative of three independent experiments. The percentage of cell viability was normalized to cells without ECM.**

| concentration of pancreatic ECM within the capsules (mg/mL) | A549 viability (%) | HEK293 viability (%) | JURKAT viability (%) |
|---|---|---|---|
| 2.0 | 100.83 ± 5.52 | 62.16 ± 7.13 | 69.56 ± 1.87 |
| 1.0 | 101.31 ± 1.67 | 86.44 ± 10.15 | 87.76 ± 0.46 |
| 0.5 | 96.12 ± 2.02 | 84.24 ± 6.81 | 93.05 ± 1.22 |
| 0.25 | 101.26 ± 1.11 | 84.51 ± 4.83 | 96.23 ± 2.17 |
| 0.125 | 104.46 ± 1.43 | 93.18 ± 4.12 | 92.10 ± 1.16 |

These results show a reduction in viability of the Jurkat and HEK293 cells when contacted with ECM at 2.0 mg/mL.. The viability depends on the cell lines, as A549 cells remain viable at this concentration. Concentrations equal or lower than 1.0 mg/L allowed a good viability for all the tested cell lines.

### 5.2. Effect on MSCs viability

The microcapsules are produced as specified in Example 7.1 (free of zwitterionic chitosan coating). The biopolymer is composed of alginate 2.5%w/w, 150 MSCs / capsule and the ECM concentration as mentioned in the first column of table 15. The starting concentration and volume of each involved components are adjusted proportionally to the procedure described in example 7.1.

MSCs viability was determined by flow cytometry, using the signal of propidium iodide (PI). PI is an intercalating agent exhibiting red fluorescence, commonly used for identifying dead cells in a population. Table 15 shows the percentages of viability of MSC by the subtraction of dead cells to the total number of cells screened.

**Table 15: MSCs viability within the capsules depending on the presence and concentration of ECM within the capsule.**

| | MSCs viability | | | |
|---|---|---|---|---|
| | D0 - day of encapsulation | D1 - day after encapsulation | D2 after encapsulation | D3 after encapsulation |
| Naked MSCs (adhering, not within capsules)(control) | 100% | 100% | 100% | 100% |
| MSCs within the capsules without ECM (reference) | NA | 62% | 58% | 55% |
| MSCs within the capsules with 1.0 mg/L of ECM | NA | 66% | 63% | 59% |
| MSCs within the capsules with 0.7 mg/L of ECM | NA | 65% | 61% | 57% |
| MSCs within capsules with 0.1 mg/L of ECM | NA | 68% | 62% | 58% |

| | | | | |
|---|---|---|---|---|
| The experiments were done with 10 000 events. | | | | |

MSCs viability was similar at all the tested ECM concentrations.

Except if otherwise stated, all the other examples were carried out with capsules comprising 0.1 mg/mL of ECM.

### 5.3 Effect of ECM on human islets encapsulated

The results are provided at tables 16 and 17.

**Table 16: Human islets viability in contact with ECM. The results are representative of three independent experiments. The percentage of Human islets viability was normalized to Human islets not within capsules.**

| | Viability (fold) |
|---|---|
| Human islets (not within capsules, controls) | 1.00 ± 0.16 |
| Human islets within capsule (reference) | 0.77 ± 0.20 |
| Human islets within capsule with 0.15 mg/ml of ECM and MSCs | 0.91 ± 0.15 |

**Table 17: stimulation index of human islets under ECM exposure**

| | SI |
|---|---|
| Human islets (not within capsules, controls) | 3.1 ± 0.99 |
| Human islets within capsule (reference) | 3.9 ± 1.50 |
| Human islets within capsule with 0.15 mg/ml of ECM and MSCs | 2.5 ± 0.52 |

### Example 7: Influence of a zwitterionic chitosan coating

The zwitterionic was composed with chitosan (PROTOSAN UP CL 114, MW 50 000-150 000 g/mol, Novamatrix) grafted with 28% (expressed as the number of moieties grafted for 100 deacetylated anhydroglucose units) of phosphorylcholine (PC) groups.

Bioactive capsules composition with human MSCs, without islets and without or with zwitterionic chitosan coating were implanted in rat (on the same animal). Histological cuts were performed in the implanted tissue and observed by microscopy after staining.

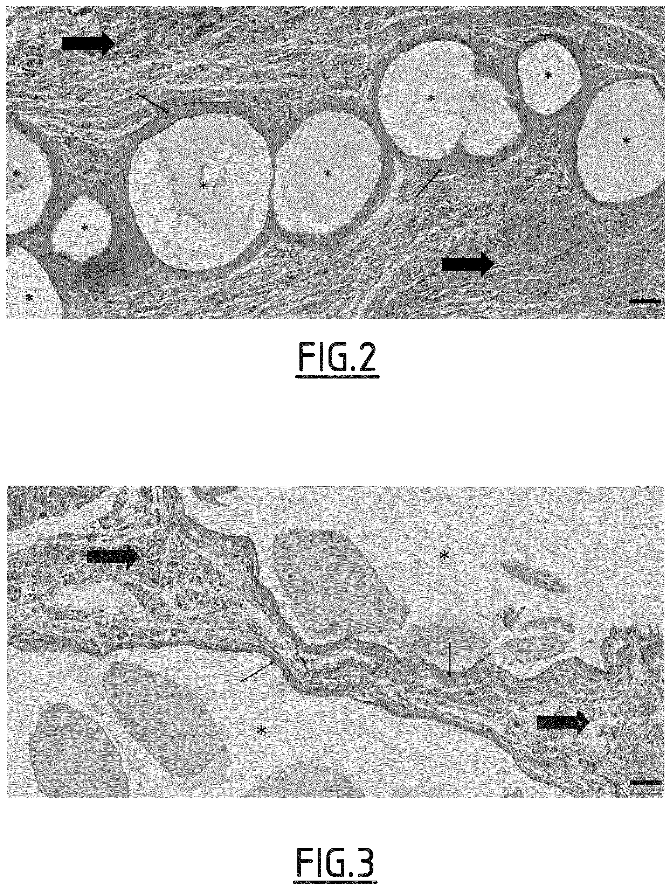

The protocol for obtaining figures 2 and 3 was:
1) Biopsy procurement: rat euthanasia one month after implantation, followed by exposure of the subcutaneous tissue and dissection of the fibrotic pocket containing the capsules
2) Fixation: 4% formaldehyde (10x biopsy volume) for 24 hours at room temperature
3) Paraffin embedding with Sakura Tissue-Tek® VIP® 6-E2
4) Microtomy: 5 µm cuts using the microtome Thermo Scientific Microm HM 340E
5) Histological staining: Haematoxylin-Eosin
6) Image acquisition: Leica SCN400 at 20x resolution

The heavy stained tissues present around the capsules devoid of zwitterionic chitosan coating illustrate a violent fibrotic response which prevents the diffusion of oxygen and nutrients in the capsule (figure 2). For the capsules coated with zwitterionic chitosan, an empty space around the capsules is observed which evidence milder fibrotic response (figure 3).

**Table 18: Viability of human MSCs with and without ZW coating.**

| | | % viability |
|---|---|---|
| Day +1 after encaps 17.01.2018 | capsule -ZW | 85.36 |
| | capsule +ZW | 76.51 |
| Day +1 after encaps 14.02.2018 | capsule -ZW | 60.48 |
| | capsule +ZW | 65.35 |
| Day +3 after encaps 16.02.2018 | capsule -ZW | 43.36 |
| | capsule +ZW | 41.13 |
| Day +7 after encaps 20.02.2018 | capsule -ZW | 21.65 |
| | capsule +ZW | 15.33 |

| | | |
|---|---|---|
| -ZW: free of zwitterionic chitosan coating +ZW: with a zwitterionic chitosan coating | | |

Except if otherwise stated, the capsules used in the other examples were free of chitosan coating.

### Example 7: Process for the preparation of capsules

A microfluidic card, in Cyclic Olefin Copolymer (COC) with micro-machined channels from 200 to 550 µm, has been developed in order to achieve the encapsulation of the cells and the cross-linking of the material mainly made of alginate. Fluids are actuated using pressure-driven pumps and valves. Capsules are produced through microfluidic flow-focusing device (MFFD). The continuous phase made of super refined soybean oil USP EP-LQ-(MH) (Croda, France), abbreviated SO, shear the dispersed phase being the bioactive material containing the islets and cells to produce periodical microcapsules. The gelation of the capsule is formed by cross linkage of calcium ions in the alginate. After forming the capsules, a co-flow SO/SO + nanoparticles of calcium starts the gelation of the capsules. Next, the capsules fall in a calcium solution to complete gelation. Eventually, capsules are washed with calcium enriched physiological serum and then stored in the adequate medium.

An optimized procedure to prepare the mixture used for the generation of microcapsules is described hereafter.

### 7.1. BIOACTIVE microcapsule

The bio-materials used for the formulation of the bioactive capsule are:
- PRONOVA SLM100 ("SLM100" hereafter) as alginate, at 2.5% w/w
- NOVATACH™ MVG GRGDSP (GRGDSP-coupled high G high MW alginate) as alginate RGD, at 0.5 mg/mL,
- Ultra-pure ECM at 0.1 mg/mL,
- Human MSCs at 150 cells/capsule,
- human or rat insulin secreting human islets as insulin-secreting cells.

To produce sterile microcapsules, buffers used in the process have been prepared from low endotoxin content products, provided by Merck Millipore (Emprove Products). All buffers have been prepared under safety cabinet and stored in sterile and low endotoxin content tanks. Aqueous buffers used are alginate buffer solution (25 mM HEPES-Na, 125 mM NaCl, pH 7.4), calcium gelling bath (25 mM HEPES-Na, 70 mM CaCI2, 30 mM NaCl, pH 7.4), calcium-enriched physiological serum buffer (10 mM HEPES-Na, 2 mM CaCI2, 150 mM NaCl, pH 7.4).
UCLouvain provided the Zwitterionic chitosan.

Batch solutions are prepared as follow:
- **SLM100 4% w/w**
   ∘ Packaging of SLM100 is 250 mg vial of lyophilised powder,
   ∘ Add 5.4 mL of water to SLM100,
   ∘ Stir 12 to 24 hours with magnetic stirrer,
   ∘ Add 600 µL of alginate buffer solution 10X,
   ∘ Stir 2 to 4 hours with magnetic stirrer to obtain an alginate solution comprising SLM100.
- **MVG GRGDSP 10 mg/mL**
   ∘ Packaging is 100 mg vial of lyophilised powder,
   ∘ Add 10 mL of alginate buffer solution 1X to MVG GRGDSP,
   ∘ Stir 12 to 24 hours with magnetic stirrer to obtain an alginate solution comprising MVG GRGDSP.
- **Ultra-pure ECM 10 mg/mL**
   ∘ Packaging is 10 mg lyophilised powder,
   ∘ Add 1 mL of alginate buffer solution 1X to ECM,
   ∘ Stir manually with pipette to obtain an alginate solution comprising ECM.
- **First aqueous solution comprising SLM100 3.65% w/w MVG GRGDSP 0.73 mg/mL, Ultrapure ECM 0.15 mg/mL** *(step a1'))*
   ∘ Add in the alginate solution comprising SLM100:
      ▪ 480 µL of alginate solution comprising MVG GRGDSP, and
      ▪ 96 µL of alginate solution comprising ECM,
   ∘ Stir 2 hours with a magnetic stirrer to obtain the first aqueous solution comprising SLM100, MVG GRGDSP and ECM.
- **Second aqueous solution comprising MSCs (MSCs 150 cells/capsule) and islets (1.5 islet/capsule)** *(step a2))*
   The number of islets to encapsulate determines the number of capsules to produce (1.5 islets/capsule) hence a volume of encapsulation solution and a number of MSCs to use.
   ∘ MSCs are delivered adherent to the cell culture support.
   ∘ Detach MSCs with trypsin 5 to 7 min *(step a2'i)),*
   ∘ Retrieve them in culture media *(step a2'i)),*
   ∘ Centrifuge 5 to 10 min at 600G *(step a2'ii)),*
   ∘ Remove supernatant, resuspend in calcium-enriched physiological serum with a pipette *(step a2'iii)),*
   ∘ Count MSCs *(step a2'iv)),*
   ∘ Needed volume of cell suspension taken *(step a2'v)),*
   ∘ Centrifuge 3 min at 150G *(step a2'vi))*
   ∘ Supernatant is removed *(step a2'vii)),*
   ∘ Suspend in 1 mL of alginate buffer solution 1X *(step a2'vii)),*
   ∘ Add the islets *(step a2'viii)),*
   ∘ Centrifuge 3 min at 150G,
   ∘ Remove a calculated volume of supernatant on the MSCs in order to reach the final encapsulation solution when the first solution will be added.
- **Encapsulation solution comprising SLM100 (2.5% w/w), MVG GRGDSP (0.5 mg/mL), Ultrapure ECM (0.1 mg/mL), MSCs (150 cells/capsule) and islets (1.5 islet/capsule)** *(step a3))*
   ∘ Add the first solution to the second solution,
   ∘ Stir manually with a gel handling pipette tip
      The encapsulation solution is ready to use.

The islets are then encapsulated on Microfluidic Flow Focusing Device (MFFD) COC card *(step b)).* Channels have a 550-µm-wide-and-deep section. The produced capsules exit the card and fall into calcium bath to crosslink and are put in calcium-enriched physiological serum 50 mL Falcon every 5 minutes. Production takes between 2 to 4 hours.
- wash *(step β))*
   ∘ Remove all the supernatant
   ∘ Transfer capsules in a new calcium-enriched physiological serum bath
   ∘ Suspend

### 7.2. zwitterionic chitosan coating

The produced capsules are then coated with zwitterionic chitosan prepared as such:
- **Zwitterionic chitosan**
   ∘ Frozen aqueous solution of zwitterionic chitosan ready to use at a concentration of 0.3 mg/mL
   ∘ Defreeze the solution
   ∘ Adjust pH to 7.4
      The zwitterionic chitosan is ready to use
- Zwitterionic chitosan coating *(step c))*
   ∘ Remove supernatant of the suspension of capsules,
   ∘ Add 1 mL of zwitterionic chitosan to 1000 capsules,
   ∘ Suspend manually with pipette for 3 minutes,
- Wash *(step β) twice)*
   ∘ Let capsules settle
   ∘ Remove the supernatant
   ∘ Add 50 mL of calcium-enriched physiological serum
   ∘ Let capsules settle
   ∘ Remove the supernatant
   ∘ Add 50 mL of calcium-enriched physiological serum

## Claims

1. Capsule comprising alginate encapsulating :
- insulin-secreting cells,
- a RGD-alginate,
- mesenchymal stem cells, and
- extracellular matrix.

2. Capsule according to claim 1, wherein the counter ions of the alginate are chosen from calcium, barium, cobalt and mixtures thereof, and preferably from calcium and mixtures of barium and calcium, and are most preferably calcium.

3. Capsule according to claim 1 or 2, wherein the insulin-secreting cells are insulin secreting human islets.

4. Capsule according to claim 3, wherein the mean number of islets per capsule is from 0.5 to 3.0, preferably from 0.8 to 2.0, most preferably from 0.9 to 1.5, 1.0 being particularly preferred.

5. Capsule according to anyone of claims 1 to 4, wherein the alginate is a M-rich alginate or a G-rich alginate.

6. Capsule according to anyone of claims 1 to 5, comprising from 0.5 to 4% by weight, preferably from 1.5 to 3.5% by weight, most preferably from 2.0 to 3.0% by weight of alginate.

7. Capsule according to anyone of claims 1 to 6, comprising:
- from 0.1 to 1.5 mg/mL, most preferably from 0.2 of 1.0 mg/mL, of RGD-alginate, versus the total volume of alginate, RGD-alginate, mesenchymal stem cells and extracellular matrix, and/or
- from 0.01 to 1.0 mg/L, most preferably from 0.05 to 0.5 mg/L, of ECM versus the total volume of alginate, RGD-alginate, mesenchymal stem cells and extracellular matrix, and/or
- from 10 to 10000 MSCs, notably from 20 to 5000 MSCs, preferably from 25 to 500 MSCs, most preferably from 50 to 150 MSCs per capsule.

8. Capsule according to anyone of claims 1 to 7, wherein the thickness of the alginate from the insulin-secreting cells to the surface of the capsule is preferably lower than 300 µm, notably lower than 250 µm, most preferably lower than 200 µm.

9. Capsule according to anyone of claims 1 to 8, having a mean diameter from 50 µm to 5 mm, preferably from 50 µm to 3 mm, to 75 to 950 µm, most preferably from 100 to 800 µm.

10. Capsule according to anyone of claims 1 to 9, having a coefficient of variation (CV) lower than 5%, typically from 3 to 4%.

11. Capsule according to anyone of claims 1 to 10, wherein the surface of the capsule is at least partly coated with a zwitterionic polymer.

12. Capsule according to claim 11, wherein the zwitterionic polymer is chitosan on which zwitterionic moieties have been grafted.

13. Process for preparing the capsule according to anyone of claims 1 to 12, comprising the steps of:
a) providing an encapsulation solution which is an aqueous solution comprising alginate, RGD-alginate, ECM, MSCs and insulin-secreting cells,
b) encapsulating insulin-secreting cells by bringing droplets of the encapsulation solution in contact with an aqueous solution comprising multivalent cations able to react with alginate, to obtain at least a capsule comprising alginate encapsulating insulin-secreting cells, the RGD-alginate, mesenchymal stem cells, and extracellular matrix,
c) optionally at least partly coating the surface of the capsule by a zwitterionic polymer.

14. Process according to claim 13, comprising between steps a) and b), a step α) consisting of forming droplets of the encapsulation solution by introducing the encapsulation solution within a Microfluidic Flow-Focusing Device or by use of centrifugal microfluidics.

15. Capsules according to anyone of claims 1 to 12 for use in the treatment of diabetes, preferably type 1 and/or insulin-treated type 2 diabetes.

## Amended claims

### Amended claims in accordance with Rule 137(2) EPC.

1. Capsule comprising alginate encapsulating :
- insulin-secreting cells,
- a RGD-alginate,
- mesenchymal stem cells, and
- extracellular matrix,
wherein the surface of the capsule is at least partly coated with a zwitterionic polymer.

2. Capsule according to claim 1, wherein the counter ions of the alginate are chosen from calcium, barium, cobalt and mixtures thereof, and preferably from calcium and mixtures of barium and calcium, and are most preferably calcium.

3. Capsule according to claim 1 or 2, wherein the insulin-secreting cells are insulin secreting human islets.

4. Capsule according to claim 3, wherein the mean number of islets per capsule is from 0.5 to 3.0, preferably from 0.8 to 2.0, most preferably from 0.9 to 1.5, 1.0 being particularly preferred.

5. Capsule according to anyone of claims 1 to 4, wherein the alginate is a M-rich alginate or a G-rich alginate.

6. Capsule according to anyone of claims 1 to 5, comprising from 0.5 to 4% by weight, preferably from 1.5 to 3.5% by weight, most preferably from 2.0 to 3.0% by weight of alginate.

7. Capsule according to anyone of claims 1 to 6, comprising:
- from 0.1 to 1.5 mg/mL, most preferably from 0.2 of 1.0 mg/mL, of RGD-alginate, versus the total volume of alginate, RGD-alginate, mesenchymal stem cells and extracellular matrix, and/or
- from 0.01 to 1.0 mg/L, most preferably from 0.05 to 0.5 mg/L, of ECM versus the total volume of alginate, RGD-alginate, mesenchymal stem cells and extracellular matrix, and/or
- from 10 to 10000 MSCs, notably from 20 to 5000 MSCs, preferably from 25 to 500 MSCs, most preferably from 50 to 150 MSCs per capsule.

8. Capsule according to anyone of claims 1 to 7, wherein the thickness of the alginate from the insulin-secreting cells to the surface of the capsule is preferably lower than 300 µm, notably lower than 250 µm, most preferably lower than 200 µm.

9. Capsule according to anyone of claims 1 to 8, having a mean diameter from 50 µm to 5 mm, preferably from 50 µm to 3 mm, to 75 to 950 µm, most preferably from 100 to 800 µm.

10. Capsule according to anyone of claims 1 to 9, having a coefficient of variation (CV) lower than 5%, typically from 3 to 4%.

11. Capsule according to anyone of claims 1 to 10, wherein the zwitterionic polymer is chitosan on which zwitterionic moieties have been grafted.

12. Process for preparing the capsule according to anyone of claims 1 to 11, comprising the steps of:
a) providing an encapsulation solution which is an aqueous solution comprising alginate, RGD-alginate, ECM, MSCs and insulin-secreting cells,
b) encapsulating insulin-secreting cells by bringing droplets of the encapsulation solution in contact with an aqueous solution comprising multivalent cations able to react with alginate, to obtain at least a capsule comprising alginate encapsulating insulin-secreting cells, the RGD-alginate, mesenchymal stem cells, and extracellular matrix,
c) at least partly coating the surface of the capsule by a zwitterionic polymer.

13. Process according to claim 12, comprising between steps a) and b), a step α) consisting of forming droplets of the encapsulation solution by introducing the encapsulation solution within a Microfluidic Flow-Focusing Device or by use of centrifugal microfluidics.

14. Capsules according to anyone of claims 1 to 11 for use in the treatment of diabetes, preferably type 1 and/or insulin-treated type 2 diabetes.
